# EUROPEAN PATENT SPECIFICATION

(11) **EP 1 587 449 B1**
(45) Date of publication and mention of the grant of the patent: **25.03.2009**
(21) Application number: 03813970.5
(22) Date of filing: 25.11.2003
(51) Int. Cl.: A61F 2/06, A61M 25/10

(54) **VARYING-DIAMETER VASCULAR IMPLANT AND BALLOON**
GEFÄSSIMPLANTAT UND BALLON MIT VERÄNDERLICHEM DURCHMESSER
IMPLANT VASCULAIRE A DIAMETRE VARIABLE ET BALLONNET

(30) Priority: 30.12.2002 IL 15375302
(43) Date of publication of application: 26.10.2005
(73) Proprietor: Neovasc Medical Ltd., Or Yehuda 60376 (IL)
(72) Inventor: BEN-MUVHAR, Shmuel, 10815 Emek Beit Shean (IL)
(74) Representative: Merrifield, Sarah Elizabeth
(86) International application number: PCT/IL2003/000996
(87) International publication number: WO 2004/058097

(56) References cited:
- EP-A- 1 254 644
- WO-A-97/17101
- US-A- 6 120 534
- US-A1- 2002 183 777
- US-B1- 6 348 066

## Description

### FIELD OF THE INVENTION

The present invention relates generally to implantable therapeutic devices, and specifically to varying-diameter intravascular implants.

### BACKGROUND OF THE INVENTION

Stent implants are commonly used in treating arterial stenoses and other unwanted constrictions of body passages. Stents typically comprise a metal coil or mesh. An arterial stent, for example, is threaded through the vascular system to the point of stenosis in an artery. When the stent is in place, it is expanded to force the artery open to the desired diameter. Typically, the stent comprises a plastic material, which is inserted using a balloon catheter into the point of stenosis in a compressed state. The stent is then expanded by inflating the balloon. An apparatus and method for securing a stent to a balloon catheter is described, for example, in U.S. Patent 6,364,870.

On the other hand, there are some procedures in which stent implants are required to constrict the diameter of a blood vessel. For example, Ruiz describes an endoluminal stent having adjustable constriction in U.S. Patent 6,120,534. The stent comprises a deformable mesh having a conical portion and a constricted region, which forms a flow-limiting constriction. The stent is delivered and deployed inside a blood vessel. The constricted region of the mesh is then selectively enlarged to adjust the flow impedance in the vessel. Ruiz describes particularly the use of his stent to reduce blood flow in the pulmonary artery, as a palliative treatment for infants having complex congenital cardiac malformations.

Other types of constricting stents and applications of such stents are described by Shalev et al. in PCT Patent Publication WO 01/72239. In particular, this publication describes the use of a flow-reducing implant in the coronary sinus, in order to promote angiogenesis in the heart tissues. The implant is inserted by catheter through a central vein, such as the jugular vein, and brought into the coronary sinus. Alternatively, the implant may be installed in one or more of the coronary veins. Once the implant is in place, it is allowed to elastically expand or it is plastically expanded using a balloon.

Examples of high-pressure balloons, traditionally used in angioplasty, and recent balloon design development, are described in an article entitled, "Applications of High-Pressure Balloons for Medical Device Industry," Medical Device and Diagnostic Industry Magazine (September 2000). Recent improvements in materials, balloon shape design, and fabrication technology include, *inter alia,* additional lengths, ultra thin walls (for minimal invasiveness and a smaller profile), varying diameters throughout the balloon length, custom shapes, and tapered ends and angles.

The specific shape of a high-pressure balloon may be demanded by the peculiarities of an anatomical site and/or the requirements of the treatment process. For example, a dog bone shaped balloon may be used to localize delivery of medication to avoid systemic intravenous administration. The ends of the balloon can be of equal or different sizes, depending on the shape of the cavity or vessel. When inflated, the ends seal off the area to be treated, and the medication is infused through a hole or series of holes in the narrower centre section of the balloon. High-pressure balloons are also used to position diagnostic devices inside vessels or body cavities for ultrasound imaging and other techniques. Rather than having a complicated steering or positioning mechanism on the end of a catheter, a high-pressure balloon can be used to either centre or offset the device, precisely positioning it as required.

EP 1254644 describes a stent and deployment arrangement including a balloon which is non-cylindrical when inflated and thereby causes the stent to assume a non-cylindrical expanded state. In this way, the stent shape can suit the geometry of the target area in a blood vessel while still pressing outward against the vessel wall over the whole length of the stent.

US 2002/0183777 describes an angioplasty catheter having a funnel-shaped opening at the distal end to receive a deflated balloon.

US 6,120,534 describes a stent with deformable constrictions for regulating blood flow.

### SUMMARY OF THE INVENTION

The present invention provides apparatus of treatment of a coronary sinus of a patient as set out in claim 1. Described herein are novel devices and methods for deploying an implant in a body passage, such as the coronary sinus, that varies in diameter over its length. In implantation of stents known inn the art, a balloon whose diameter is roughly uniform over its length is typically used. Therefore, if the diameter of the body passage varies over the length of the stent, the end of the stent in the wider area of the passage may be insufficiently expanded, so that the stent is not securely anchored. Alternatively, the opposite end of the stent, in the narrower area of the body passage, may be expanded substantially beyond the natural diameter of the passage, causing strain on the tissue.

In embodiments of the present invention, on the other hand, the balloon that is used to expand the implant has a diameter that varies over its length, in such a way as to roughly match the varying diameter of the body passage. When the implant is in place within the body passage, the balloon is inflated to plastically expand the implant, so that the expanded diameter of the implant roughly matches the full diameter of the body passage at two or more points, typically at both ends of the implant. (In the case of a constricting implant, as may be used in the cardiac sinus in order to partially constrict the flow of blood therethrough, a part of the implant, typically a central part, may remain unexpanded.) As a result, the implant is anchored securely in place, without undue strain on the walls of the body passage.

The implant and balloon and method of inserting them described herein are particularly useful for restricting blood flow in the coronary sinus, as described in the above-mentioned PCT publication and in U.S. Patent Application 09/534,968, which is assigned to the assignee of the present patent application and whose disclosure is incorporated herein by reference. The principles of the present invention, however, may be similarly used in deploying implants within other varying-diameter veins and arteries, as well as in other medical applications.

There is therefore described herein a method for deploying an expandable implant in a body passage of varying diameter, including:
selecting a balloon having a radial dimension that varies, when the balloon is inflated, in accordance with the varying diameter of the body passage;
inserting the balloon, in a deflated state, into the body passage, with the expandable implant fitted radially around the balloon; and
inflating the balloon so as to cause the implant to open, responsively to the varying radial dimension of the balloon, into an expanded shape that approximately matches the varying diameter of the body passage, thus anchoring the implant in the body passage.

Typically, the method includes attaching the balloon to a catheter and passing the balloon into the body passage using the catheter.

The body passage is a coronary sinus of a patient, and passing the balloon includes:
guiding the catheter through a vascular path into a right atrium of the patient; and
steering the catheter within the right atrium so as to position the balloon and the implant in the coronary sinus.

Typically, the selected balloon has distal and proximal ends, and the radial dimension of the distal end is substantially smaller than the radial dimension of the proximal end. In one example, the selected balloon has a generally conical profile.

In other embodiments, the selected balloon includes a proximal segment having a first diameter and a distal segment having a second diameter, which is substantially smaller than the first diameter. In one of these embodiments, at least one of the segments terminates in a bulb, having a third diameter that is greater than the diameter of the at least one of the segments. In another embodiment, the selected balloon includes a neck intermediate the proximal and distal segments, the neck having a third diameter that is less than the second diameter.

The method may also include deflating the balloon after the implant has opened, drawing the deflated balloon in a distal direction into a tubular accessory, and withdrawing the accessory, containing the balloon, from the body passage. Drawing the deflated balloon in the distal direction may include widening a distal end of the tubular accessory in order to receive the balloon.

Additionally or alternatively, selecting the balloon may include measuring the diameter of the body passage at multiple points along the passage, and choosing the balloon from among a selection of available balloons, so as to fit the radial dimension of the balloon to the measured diameter of the body passage.

When the body passage is a coronary sinus of a patient, choosing the balloon includes fitting the balloon to a widening region of the coronary sinus adjacent to a right atrium of the patient. Typically, the implant includes a constriction, and inflating the balloon includes expanding the implant to match the varying diameter of the coronary sinus except at the constriction, so as to inhibit a flow of blood through the coronary sinus.

There is also described herein apparatus for treatment of a body passage of varying diameter, including:
a balloon having a radial dimension that varies, when the balloon is inflated, in accordance with the varying diameter of the body passage; and
an expandable implant, fitted radially around the balloon, so that when the balloon is inflated within the body passage, the implant opens, responsively to the varying radial dimension of the balloon, into an expanded shape that approximately matches the varying diameter of the body passage, thus anchoring the implant in the body passage. Typically, the apparatus includes a catheter, which is adapted to deploy the balloon and implant in the body passage.

Typically, the balloon is one of a plurality of balloons having different radial dimensions, which are selectable for insertion into the body passage depending upon a measured diameter of the body passage at multiple points along the passage.

The present invention will be more fully understood from the following detailed description of the embodiments thereof, taken together with the drawings in which:

### BRIEF DESCRIPTION OF THE DRAWINGS

Fig. 1A is a schematic, pictorial view of an exemplary implantable device, in a non-expanded position, in accordance with an embodiment of the present invention;
Fig. 1B is a schematic, pictorial view of the exemplary implantable device shown in Fig. 1A, in an expanded position;
Fig. 2 is a schematic, pictorial view of an exemplary stent balloon, but is not an embodiment of the present invention;
Fig. 3 is a schematic view of the vascular path to a human heart having a coronary sinus;
Fig. 4 is a detailed schematic view of the coronary sinus following expansion of an implantable device by the balloon shown in Fig. 2, in accordance with an embodiment of the present invention;
Figs. 5-7 are schematic, pictorial views of exemplary stent balloons, figures 6 and 7 being in accordance with alternative embodiments of the present invention;
Fig. 8 is a schematic, pictorial view of a deflated balloon inside a stent and an accessory used in removing the deflated balloon from the stent, in accordance with an embodiment of the present invention; and
Figs. 9A and 9B are schematic, detail views showing steps in a process of removing a deflated balloon from a stent, in accordance with an embodiment of the present invention.

### DETAILED DESCRIPTION OF EMBODIMENTS

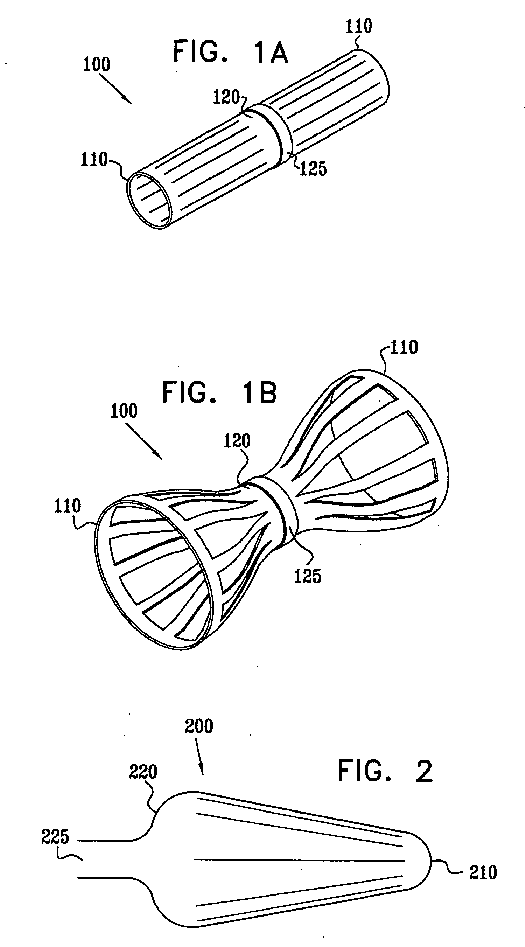

Reference is now made to Figs. 1A and 1B, which are schematic, pictorial views of an exemplary implantable device 100, in a constricted state and an expanded state, respectively, in accordance with an embodiment of the present invention. Device 100 is adapted for use particularly in restricting blood flow through the coronary sinus, as described in the above-mentioned PCT Publication WO 01/72239 and U.S. Patent Application 09/534,968. Alternatively, devices in accordance with the principles of the present invention may be implanted elsewhere in the vascular system, as well as in other body passages. For the sake of simplicity and clarity, however, and not limitation, embodiments of the present invention are described hereinbelow with reference to implantation of flow-constricting devices in blood vessels of varying diameter, such as the coronary sinus.

Device 100 is of general tubular construction with two expandable ends 110 and a central section 120. Further alternatively or additionally, device 100 may comprise a mesh or coil, as is known in the art. Device 100 comprises a deformable material, such as a suitable metal or plastic, as is known in the art of implantable devices, which is sufficiently flexible to be expanded by inflation of a balloon (shown in Fig. 2), but strong enough to hold its shape when it is deployed and expanded within a body passage, in the manner of stents known in the art. Furthermore, the shape of device 100, combined with its flexibility, enables the device to be deployed in compact form, as shown in Fig. 1A, and subsequently expanded, as shown in Fig. 1B, either partially or completely, within the coronary sinus. A non-expandable constricting element 125 may be attached around a central section 120 of device 100, in order to ensure that the central section remains constricted, as shown in Fig. 1B.

A flexible sleeve (not shown) may be fixed around or within device 100, in order to prevent blood from flowing through the openings in the sides of the device when it is implanted, so that substantially all the blood flows through central section 120. Typically, the sleeve comprises a biocompatible fabric such as Gore-Tex or Dacron, which is stitched or otherwise fastened to device 100. Alternatively, other sleeve materials may be used, such as thin plastic or rubber materials. Constricting element 125 is fitted around the sleeve, over central section 120. As can be seen in Fig. 1B, the effect of the constricting element is to maintain a predetermined reduced diameter of device 100 in the region of central section 120, defining a lumen with a constricted central section diameter. Constricting element 125 may comprise a closed ring, made of metal or plastic, or it may alternatively comprise a thread.

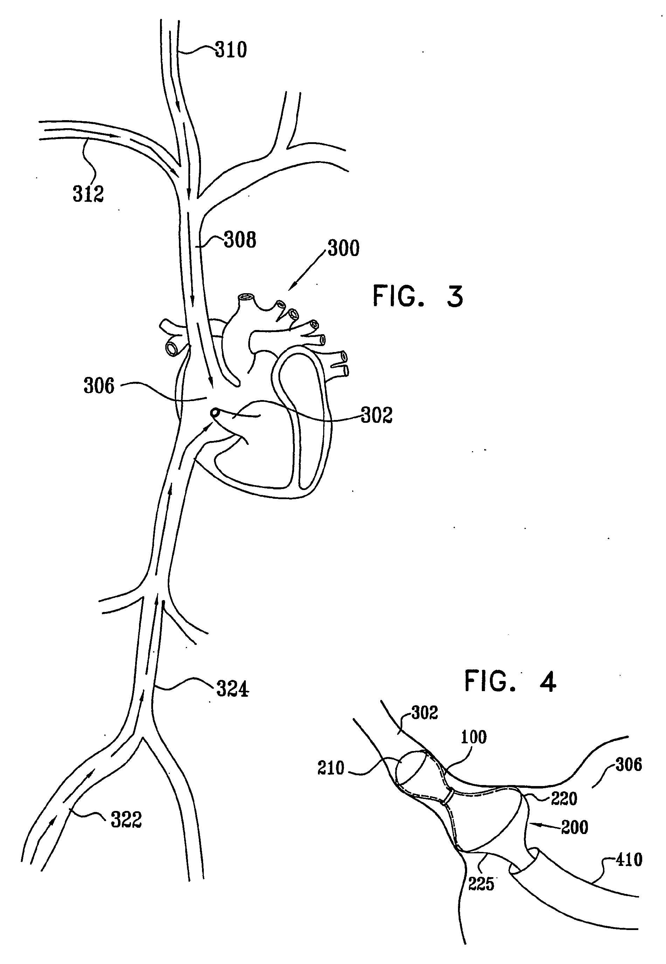

Reference is now made to Fig. 2, which is a schematic pictorial view of an exemplary high pressure stent balloon 200, used to expand device 100. Balloon 200 has a generally conical shape, having a blunt, narrowed distal end 210 and a widened proximal end 220. The balloon terminates in a taper 225, which forms a continuation of the channel portion of a catheter (shown in Fig. 4), through which the balloon is inflated and deflated. Balloon 200 typically comprises a high-pressure, non-elastic material, as is known in the art, which is designed to apply an outward radial force when inflated, as described in the above-mentioned article from Medical Device & *Diagnostic Industry Magazine.* Generally, device 100 is deployed into a body passage with deflated balloon 200 contained concentrically within it. The shape of balloon 200 is adapted so that when balloon inflates, it expands device 100 and positions it within a preselected varying-diameter body passage, as is discussed hereinbelow.

Balloon 200 is typically fabricated from materials such as polyethlylene tererphthalate (PET) or nylon. Some considerations for fabricating balloon 200 using these materials include: high tensile strength, allowing high operating pressures; thin balloon wall formation, allowing precise balloon shape and low profile; and low elongation (otherwise known as "low compliance"). The latter consideration ensures that balloon 200, when fully pressurized, exhibits relatively unchanging dimensions, ensuring that device 100 is not uncontrollably over-expanded in a body passage. Low elongation also means that balloon 200 will not over-expand at either end of device 100 and that the expansion force of the balloon is directed generally radially to expand device 100 substantially against the walls of the body passage.

Reference is now made to Fig. 3, which is a schematic view of vascular paths to a human heart 300 having a coronary sinus 302. Coronary sinus 302 comprises a junction of three major cardiac veins (not shown), and becomes progressively wider as it empties into a right atrium 306. The diameter of coronary sinus 302 increases as it opens out into right atrium 306.

To implant device 100, the device is passed through the vascular system to a preselected position in coronary sinus 302, using a suitable percutaneous catheter (shown in Fig. 4). Suitable methods of catheterization for this purpose are known in the art. During the insertion procedure, device 100 is maintained in the non-expanded configuration shown in Fig. 1A, so that its outer diameter is substantially smaller than the blood vessels through which it must pass, allowing the physician operating the catheter to pass the device through the blood vessels. Typically, the physician inserts the catheter through a jugular vein 310 or a subclavian vein 312, and then guides the catheter into a right atrium 306 via a superior vena cava 308. Another insertion point is through a femoral vein 322, and the catheter is then guided to an inferior vena cava 324 and into right atrium 306. Once in right atrium 306, the physician steers the catheter through a sharp bend in order to guide device 100 into coronary sinus 302.

Reference is now made to Fig. 4, which is a detailed schematic view of coronary sinus 302 following expansion of device 100 by balloon 200, in accordance with an embodiment of the present invention. A catheter 410 is used, as described hereinabove, to position the device and balloon in coronary sinus 302 via right atrium 306. Balloon 200 is then inflated, via catheter 410, and assumes a general shape as shown in the figure. The physician may choose the shape of balloon 200 in advance, so as to optimally match the given dimensions of the coronary sinus of the patient in question. These dimensions may be determined, for example, by taking fluoroscopic images while injecting a contrast agent into the coronary sinus, as is known in the art.

When balloon 200 is inflated, it applies a radial force to plastically expand device 100 against the walls of coronary sinus 302. As shown in the figure, due to the varying diameter of balloon 200, the distal end of device 100 is only partially expanded, whereas the proximal end of device 100 is more completely expanded, reflecting the varying diameter of coronary sinus 302. As previously noted, balloon 200 does not over-expand at either end of device 100. Distal end 210 of balloon may protrude slightly from the distal end of device 100. In a similar fashion, widened proximal end 220 and the taper 225 of balloon 200 may protrude from the proximal end of device 100. Because the shape of device 100 is fit to the natural shape of the coronary sinus, both the distal and proximal ends of the device press outward against the wall of the coronary sinus with approximately equal force. Thus, device 100 is securely anchored in place, without exerting excessive pressure against the wall of the coronary sinus at any point. Central section 120, however, remains constricted due to the presence of constricting element 125 or other means provided for this purpose.

Once device 100 is satisfactorily positioned and expanded, balloon 200 is deflated and withdrawn from device 100. Catheter 410 and balloon 200 are then withdrawn from the body. Device 100 remains in place to restrict the flow of blood through coronary sinus 302. As noted above, this flow restriction increases the blood pressure in the coronary veins, thereby fostering angiogenesis. Device 100 may be left in place indefinitely, in substantially the form shown in Fig. 4. Alternatively, it may be desirable in some cases to eliminate the flow restriction caused by the device. In such cases, a catheter with a suitable cutting tool may be inserted percutaneously to the location of the device, and the cutting tool may then be used to cut constricting element 125 or central section 120. A balloon, such as balloon 200, may then be reinserted via catheter into device 100 and the balloon may then be inflated in order to open section 120.

Although in the examples described above, device 100 and balloon 200 are shown to have certain particular shapes, alternative shapes and forms of these elements, which will be apparent to those skilled in the art, are considered to be within the scope of the present invention. Similarly, balloons of the general type described above may be used to deliver not only device 100, but also other implantable devices for implantation in other body passages of variable diameter, as are otherwise known in the art. Furthermore, although the catheter shown here provides a convenient' means for delivering implantable devices in accordance with the present invention, balloons in accordance with the present invention may also be used in conjunction with other means for implant deployment, including both minimally invasive (typically percutaneous) and invasive (i.e., surgical) types.

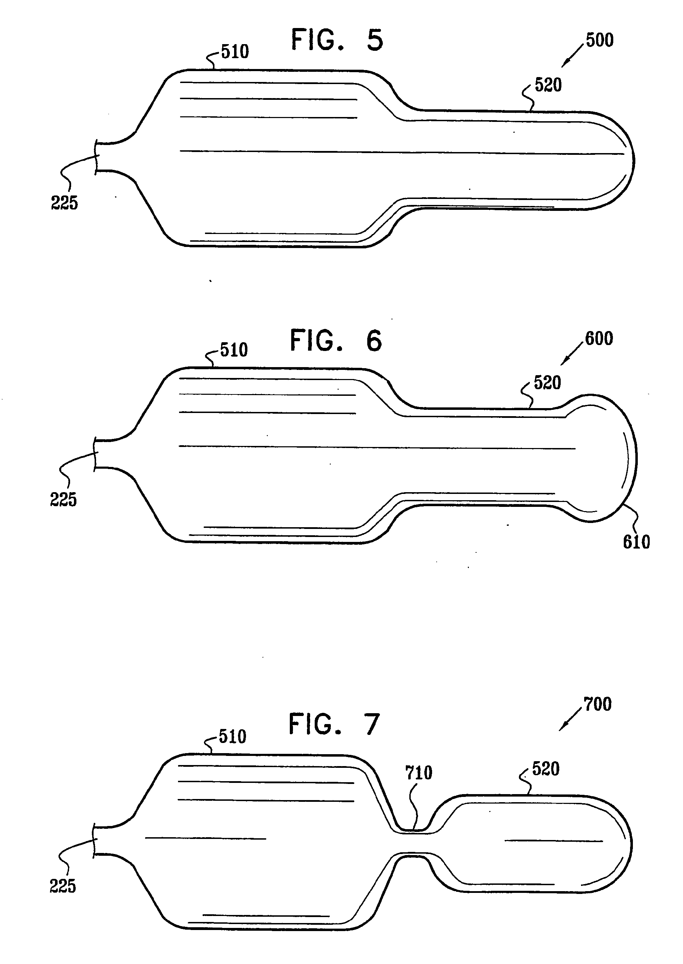

For example, Figs. 5-7 are schematic, pictorial views of balloons 500, 600 and 700, which may be used in place of balloon 200, figures 6 and 7 being in accordance with alternative embodiments of the present invention. Instead of the generally conical profile of balloon 200, these alternative balloons comprise a broad proximal segment 510 and a narrow distal segment 520. The proximal and distal segments are generally cylindrical, and have different, respective diameters. Alternatively, the proximal and distal segments may have trapezoidal profiles. For stent implantation in the coronary sinus, these balloons are typically about 30 mm long, and have diameters of about 10 mm in the broad segment and 7 mm in the narrow segment. Alternatively, larger or smaller dimensions may be used, depending on application requirements and physiological characteristics of the patient.

In balloon 600, narrow segment 520 terminates distally in a bulb 610, which is broader than the narrow segment. For example, if narrow segment 520 is 7 mm in diameter, bulb 610 may have a diameter of about 8 mm. The bulb helps to open the upstream end 110 of the stent in order to anchor the stent more securely in the coronary sinus (or other body passage). Additionally or alternatively, broad segment 510 may terminate proximally in a similar sort of a bulb.

Balloon 700 comprises a narrow neck 710 between segments 510 and 520. Typically, the neck is about 3 mm in diameter, although smaller or larger dimensions may also be used. Neck 710 fits inside central section 120 of stent 100 during inflation of the stent. It thus prevents balloon 700 from exerting pressure against non-expandable constricting element 125, and is also useful in facilitating removal of the balloon from the stent after completion of the stent implantation procedure.

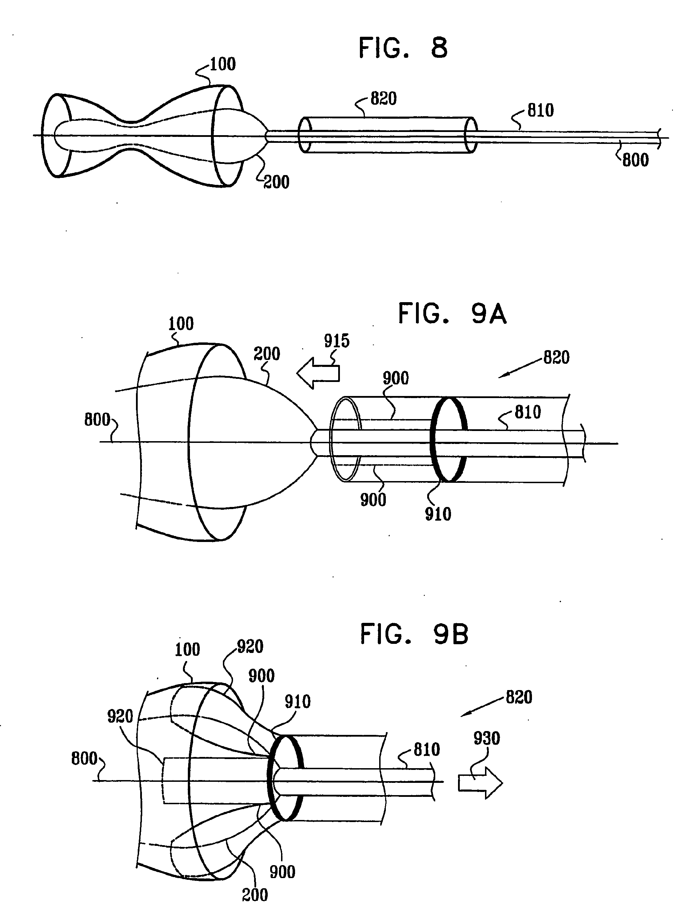

Fig. 8 is a schematic, pictorial illustration showing the use of a tubular accessory 820 in removing balloon 200 from the body, in accordance with an embodiment of the present invention. In this embodiment, an operator, typically a physician, has inserted a guide wire 800 through a patient's vascular system into the coronary sinus, using techniques known in the art. Stent 100 and balloon 200 have been passed over wire 800 into the coronary sinus, and balloon 200 has been inflated in order to expand the stent to the proper dimensions. The balloon has an annular cross-section, in order to fit over wire 800, and is inflated and deflated via an annular tube 810. At the stage of the procedure pictured in Fig. 8, balloon 200 has been deflated (likewise via tube 810), and is now to be withdrawn over wire 800 from the patient's body by pulling tube 810 in the proximal direction, out of the body.

The inventors have found that under these circumstances, it is sometimes difficult to extract balloon 200 from stent 100 and through the vascular system. Therefore, to facilitate extraction of the balloon, the operator inserts accessory 820 over wire 800 to a position just proximal of balloon 200, and then draws the balloon in the proximal direction into the accessory. Once the balloon is held inside accessory 820, the accessory containing the balloon can be withdrawn easily from the body. Similar sorts of accessories and methods may be used for inserting and extracting a balloon over other sorts of guides, such as a "monorail" guide, as is known in the art.

For these purposes, accessory 820 typically comprises a tube of small diameter, for example, about 2.8 mm, with a length of about 500 mm. The tube should be flexible enough to pass through the vascular system, but stiff enough so as not to deform significantly when balloon 200 is pulled inside it. Accessory 820 may comprise, for example, polyurethane or another biocompatible plastic material, with a wall thickness of about 0.4 mm. An additional catheter or other insertion tube (not shown in the figures) may be attached to the proximal end of accessory 820, for use in advancing the accessory into place adjacent to balloon 200, and then pulling the accessory and balloon out of the body.

Similar techniques and accessories may be used in inserting and removing balloons of other shapes, such as those shown in Figs. 5-7.

Figs. 9A and 9B schematically show details of the distal end of accessory 820 and its use in capturing balloon 200, in accordance with an embodiment of the present invention. In this embodiment, the distal end of accessory 820 is scored or perforated along score lines 900. The score lines are designed to rip open under sufficient outward radial force. A stiffening ring 910 limits the extent of the rip to a predetermined length from the distal end of the accessory, typically about 3.5 mm. Ring 910 may comprise metal or another radiopaque material, so that the location of accessory 820 is visible under X-ray imaging.

In operation, accessory 820 is advanced in the distal direction, as shown by an arrow 915 in Fig. 9A, until the scored, distal end of the accessory slides inside the expanded proximal end of stent 100. If balloon 200 is sufficiently flaccid at this point, it will be possible to draw the balloon into accessory 820 simply by pulling tube 810 in the proximal direction, as indicated by an arrow 930 in Fig. 9B. If there is residual pressure in the balloon, however, or inherent stiffness of the balloon material, the balloon may tear score lines 900, causing the distal end of accessory 820 to widen by opening into multiple flaps 920. These flaps widen out to create a funnel structure at the distal end of the accessory. This structure may be supported radially by stent 100, as shown in the figure. The funnel aids in compressing the balloon gradually as it is pulled in the direction of arrow 930, so that the balloon slides smoothly into accessory 820. Other means for widening the distal end of accessory 820 may alternatively be provided, as will be apparent to those skilled in the art.

It will be appreciated that the embodiments described above are cited by way of example, and that the present invention is not limited to what has been particularly shown and described hereinabove. Rather, the scope of the present invention includes both combinations and subcombinations of the various features described hereinabove, as well as variations and modifications thereof which would occur to persons skilled in the art upon reading the foregoing description and which are not disclosed in the prior art.

## Claims

1. Apparatus for treatment of a coronary sinus (302) of a patient, which has a widening region adjacent to a right atrium (306) of the patient, the apparatus comprising:
a balloon (600,700), having a radial dimension that varies when the balloon is inflated; and
an expandable implant (100), having distal and proximal ends (110) and a central section (120) intermediate the distal and proximal ends, wherein the implant is fitted radially around the balloon so that when the balloon is inflated within the coronary sinus, the implant opens, responsively to the varying radial dimension of the balloon, into an expanded shape,
wherein the balloon comprises:
a proximal segment (510), for placement adjacent to the right atrium during expansion of the balloon, the proximal segment having a first diameter when expanded that approximately matches the diameter of the widening region, thus anchoring the proximal end of the implant in the widening region;
a distal segment (520) having a second diameter when expanded that is substantially smaller than the first diameter and approximately matches an internal diameter of the coronary sinus, thus anchoring the distal end of the implant inside the coronary sinus; and
a neck (520,710) intermediate the proximal and distal segments, the neck having a third diameter that is sufficiently less than the second diameter when expanded so that the central section of the implant remains constricted and does not contact the coronary sinus.

2. The apparatus according to claim 1, and comprising a catheter (410), which is adapted to deploy the balloon (600,700) and implant (100) in the body passage.

3. The apparatus according to claim 2, wherein the catheter (410) is adapted to be guided through a vascular path into the right atrium of the patient and to be steered within the right atrium, so as to position the balloon (600,700) and the implant (100) in the coronary sinus.

4. The apparatus according to claim 1, and comprising a tubular accessory (820), which is adapted to be positioned at a location distal to the implant (100), and is operative, after the implant (100) has opened and the balloon (600,700) has been deflated, to receive the deflated balloon (600, 700) inside the accessory (820) and to contain the balloon (600, 700) while the accessory (820) is withdrawn from the body passage.

5. The apparatus according to claim 4, wherein the accessory (820) comprises a distal end, which is adapted to widen in order to receive the balloon (600,700).

6. The apparatus according to any of claims 1-5, wherein the balloon (600,700) is one of a plurality of balloons having different radial dimensions, which are selectable for insertion into the coronary sinus depending upon a measured diameter of the coronary sinus at multiple points along the passage.

7. The apparatus according to any of claims 1-5, wherein the constricted central section (120) of the implant (100) is configured so as to inhibit a flow of blood through the coronary sinus.

## Patentansprüche

1. Vorrichtung zur Behandlung eines Koronarsinus (302) eines Patienten, welcher einen Aufweitungsbereich benachbart zu einem rechten Vorhof (306) des Patienten aufweist, wobei die Vorrichtung umfasst:
- einen Ballon (600, 700), welcher eine radiale Abmessung aufweist, die variiert, wenn der Ballon aufgeblasen wird; und
- ein expandierbares Implantat (100), welches ein distales und ein proximales Ende (110) und einen zentralen Abschnitt (120) zwischen dem distalen und dem proximalen Ende aufweist, wobei das Implantat radial um den Ballon herum angebracht ist, so dass, wenn der Ballon innerhalb des Koronarsinus aufgeblasen wird, sich das Implantat auf die variierende Abmessung des Ballons reagierend in eine expandierte Form öffnet,
wobei der Ballon umfasst:
- einen proximalen Abschnitt (510) für eine Anordnung benachbart zu dem rechten Vorhof während einer Expansion des Ballons, wobei der proximale Abschnitt, wenn er expandiert ist, einen ersten Durchmesser aufweist, welcher etwa gleich dem Durchmesser des Aufweitungsbereiches ist, wodurch er das proximale Ende des Implantats in dem Aufweitungsbereich verankert;
- einen distalen Abschnitt (520), welcher, wenn er expandiert ist, einen zweiten Durchmesser aufweist, welcher im Wesentlichen kleiner als der erste Durchmesser und etwa gleich einem Innendurchmesser des Koronarsinus ist, so dass er das distale Ende des Implantats innerhalb des Koronarsinus verankert; und
- einen Hals (520, 710) zwischen dem proximalen und dem distalen Abschnitt liegend, wobei der Hals einen dritten Durchmesser aufweist, welcher hinreichend kleiner als der zweite Durchmesser ist, wenn er expandiert ist, so dass der zentrale Abschnitt des Implantats verengt bleibt und nicht den Koronarsinus berührt.

2. Vorrichtung nach Anspruch 1 und umfassend einen Katheter (410), welcher dazu geeignet ist, den Ballon (600, 700) und das Implantat (100) in der Körperpassage anzuwenden.

3. Vorrichtung nach Anspruch 2, wobei der Katheter (410) dazu geeignet ist, durch einen Gefäßweg in den rechten Vorhof des Patienten geführt und innerhalb des rechten Vorhofs gelenkt zu werden, um den Ballon (600, 700) und das Implantat (100) in dem Koronarsinus zu positionieren.

4. Vorrichtung nach Anspruch 1 und umfassend ein röhrenförmiges Zusatzteil (820), welches geeignet ist, an einem im Bezug auf das Implantat (100) distalen Ort positioniert zu werden, und welches betriebsfähig ist, nachdem das Implantat (100) sich geöffnet hat und der Ballon (600, 700) entleert worden ist, den entleerten Ballon (600, 700) ins Innere des Zusatzteils (820) aufzunehmen und den Ballon (600, 700) zu umfassen, während das Zusatzteil (820) aus der Körperpassage herausgezogen wird.

5. Vorrichtung nach Anspruch 4, wobei das Zusatzteil (820) ein distales Ende umfasst, welches dazu geeignet ist, sich aufzuweiten, um den Ballon (600, 700) aufzunehmen.

6. Vorrichtung nach einem der Ansprüche 1 bis 5, wobei der Ballon (600, 700) einer von einer Mehrzahl von Ballons ist, welche verschiedene radiale Abmessungen aufweisen, welche zum Einführen in den Koronarsinus in Abhängigkeit eines gemessenen Durchmessers des Koronarsinus an mehreren Punkten entlang der Passage auswählbar sind.

7. Vorrichtung nach einem der Ansprüche 1 bis 5, wobei der verengte zentrale Abschnitt (120) des Implantats (100) gestaltet ist, um eine Blutströmung durch den Koronarsinus zu verhindern.

## Revendications

1. Appareil pour le traitement d'un sinus coronaire (302) d'un patient, qui présente une région d'élargissement adjacente à l'oreillette droite (306) du patient, l'appareil comportant :
un ballonnet (600, 700) ayant une dimension radiale qui varie lorsque le ballonnet est gonflé ; et
un implant expansible (100) ayant des extrémités distale et proximale (110) et une section centrale (120) entre les extrémités distale et proximale, l'implant étant ajusté radialement autour du ballonnet afin que, lorsque le ballonnet est gonflé dans le sinus coronaire, l'implant s'ouvre, en réponse à la dimension radiale qui varie du ballonnet, pour prendre une forme expansée,
dans lequel le ballonnet comporte :
un segment proximal (510) destiné à être placé à proximité immédiate de l'oreillette droite pendant l'expansion du ballonnet, le segment proximal ayant un premier diamètre lorsqu'il est expansé, qui correspond approximativement au diamètre de la région d'élargissement, ancrant ainsi l'extrémité proximale de l'implant dans la région d'élargissement ;
un segment distal (520) ayant un second diamètre lorsqu'il est expansé, qui est sensiblement inférieur au premier diamètre et qui correspond approximativement au diamètre interne du sinus coronaire, ancrant ainsi l'extrémité distale de l'implant à l'intérieur du sinus coronaire ; et
un col (520, 710) entre les segments proximal et distal, le col ayant un troisième diamètre qui est suffisamment inférieur au deuxième diamètre lorsqu'il est expansé pour que la section centrale de l'implant reste resserrée et n'entre pas en contact avec le sinus coronaire.

2. Appareil selon la revendication 1, comportant un cathéter (410) qui est conçu pour déployer le ballonnet (600, 700) et l'implant (100) dans le passage corporel.

3. Appareil selon la revendication 2, dans lequel le cathéter (410) est conçu pour être guidé par une trajectoire vasculaire jusque dans l'oreillette droite du patient et pour être dirigé à l'intérieur de l'oreillette droite, afin de positionner le ballonnet (600, 700) et l'implant (100) dans le sinus coronaire.

4. Appareil selon la revendication 1, comportant un accessoire tubulaire (820) qui est conçu pour être positionné en un emplacement distal par rapport à l'implant (100), et qui peut être mis en oeuvre, après que l'implant (100) a été ouvert et que le ballonnet (600, 700) a été dégonflé, pour recevoir le ballonnet dégonflé (600, 700) à l'intérieur de l'accessoire (820) et pour contenir le ballonnet (600, 700) tandis que l'accessoire (820) est retiré du passage corporel.

5. Appareil selon la revendication 4, dans lequel l'accessoire (820) comporte une extrémité distale qui est conçue pour s'élargir afin de recevoir le ballonnet (600, 700).

6. Appareil selon l'une quelconque des revendications 1 à 5, dans lequel le ballonnet (600, 700) est l'un de plusieurs ballonnets ayant des dimensions radiales différentes, qui peuvent être sélectionnés pour une insertion dans le sinus coronaire suivant un diamètre mesuré du sinus coronaire en de multiples points le long du passage.

7. Appareil selon l'une quelconque des revendications 1 à 5, dans lequel la section centrale resserrée (120) de l'implant (100) est configurée pour empêcher un écoulement du sang à travers le sinus coronaire.
